# EUROPEAN PATENT SPECIFICATION

(11) **EP 2 360 494 B1**
(45) Date of publication and mention of the grant of the patent: **13.01.2016**
(21) Application number: 11154405.2
(22) Date of filing: 14.02.2011
(51) Int. Cl.: G01T 1/29

(54) **A high energy radiation imaging device**
Bildaufnahmevorrichtung für hochenergetischer Strahlung
Dispositif d'imagerie par radiation à haute énergie

(30) Priority: 15.02.2010 NL 2004248
(43) Date of publication of application: 24.08.2011
(73) Proprietor: Milabs B.V., 3584 CX Utrecht (NL)
(72) Inventor: Beekman, Frederik Johannes, 3584 CG, Utrecht (NL)
(74) Representative: EP&C

(56) References cited:
- WO-A2-2007/105942
- US-B2- 7 145 153

## Description

The present invention relates to a focused pinhole high energy radiation imaging device according to the preamble of claim 1, as described in US 7,145,153.

Such devices are used a. o. for small animal imaging for biomedical research, but also for other purposes such as clinical imaging and non-destructive testing.

Herein, a focused pinhole collimator has a plurality of pinholes, each pinhole having an individual field-of-view, wherein said pinholes are constructed and arranged so as to establish a common overlap of their individual fields of view, said overlap defining the focus volume. Furthermore, "rotational symmetry" has its usual meaning of shape invariance after a certain amount of rotation, around the first axis.

A known device of the type mentioned above is the U-SPECT. This device has a triangular detector surface, with a collimator consisting of a cylindrical pinhole wall mounted therein, the pinholes of which are all focused to a focus volume, or central field-of-view (CFOV), i.e. the individual fields-of-view all overlap in said CFOV. Inside the collimator there is an object space for an object to be examined.

A problem with this known device is that it is still a compromise between sensitivity, resolution of the images and space for the object, i.e. dimensions of the total device. In particular, only a relatively small part of the object space can be used, i.e. imaged, in particular only the focus volume. In order to scan objects larger than the focus volume, a much larger object space is needed, and if the focus volume is made larger, there results a loss in sensitivity and/or resolution.

It is an object of the present invention to provide a device according to the preamble, that has an improved resolution and/or sensitivity when imaging, such as by an increase in magnification factor.

Another object of the invention is to provide a device according to the preamble, that has a more flexible and efficient use of the object space when imaging.

Another, more general object of the invention is to provide an alternative device according to the preamble.

At least a part of these objects is achieved with a device according to claim 1, which is characterized in that the collimator is arranged and designed in such a way that the focus volume is located eccentrically with respect to said first axis. In other words, the pinholes in the collimator are designed and arranged in said collimator such that the focus volume is located eccentrically with respect to said first axis. Contrarily, the prior art device has a focus volume which is centered with respect to the central axis of rotation. The design according to the present invention has at least the following advantages.

Note that the first axis extends through a center of gravity, i.e. geometrical center, of the object space, the first axis being an axis of rotational symmetry of course only in case of a rotationally symmetric object space.

The focus volume is closer to at least a number of pinholes, which allows to image with a greater sensitivity, which roughly has an inverse square dependence on distance. Since at least a number of pinholes is closer to the object, it is relatively easy to obtain a larger image magnification factor, and also a better image resolution. One can also say that, with the same detector area, relatively more image magnification is possible.

Note that the opening angle of the pinholes which are closer to the focus volume can be taken larger than the opening angle of pinholes further away, at least in the case of a circular focus volume. In the present invention, this can be a characteristic of some collimators. However, this is only desirable if one wants no overlap of projections, or a similar amount of overlap, and it is certainly possible to provide a collimator with equal pinholes, providing e.g. a non-circular focus volume, or varying overlap of projections.

The present device is suitable for use with high energy radiation emitted by the object to be imaged, such as by means of radioactive tracers. In particular X-rays and more in particular gamma radiation are used. Such high energy radiation offers a useful range of radiation, having high penetration power.

In this application, and as generally understood in the art, a field of view of a pinhole corresponds to a solid angle that is imageable by the pinhole, in other words the part of three-dimensional space that can be seen by the detector through the pinhole.

The central field of view (CFOV) or focus volume, which terms are used interchangeably here, can be defined as the volume of the space that can be seen by all pinholes (of the collimator) as a whole, i.e. the overlap of all fields of view. In conventional pinhole systems, at least two pinholes with their individual fields of view are required to define a CFOV or focus volume.

Of course, it might happen that, due to production tolerance or the like, some pinhole of a prior art device does not "see" exactly the same (focus) volume, and could be called off-focus or eccentric. However, this is still in contrast with the present invention, where each pinhole should have a field of view that is designed to overlap with the other fields-of-view in a focus volume that is shifted with respect to the first axis.

The collimator has its usual function of selecting certain directions of the radiation emitted by the object to be imaged onto the detector. True and practical lenses do not yet exist for X-rays or gamma radiation, but in order to select, collimators of the present device use pinholes that transmit only radiation coming from certain directions, i.e. from their individual field-of-view, often a true cone or a pyramid-like cone. These fields-of-view are imaged onto the detector surface(s), providing as many images as there are pinholes. Suitable image reconstruction techniques can then be employed to reconstruct the isotope distribution in the object.

Such collimators often comprise thick slabs of heavy, dense metals, such as lead, tungsten, gold, iridium or (depleted) uranium, in order to block unwanted radiation. The collimator(s), together with other shielding parts to prevent radiation leaks onto the detector(s) or into the environment, are often very heavy. Therefore, a compact device is very advantageous as to weight, handling and use of natural resources (and therefore price).

The detector device may comprise several detector units, which are more or less independent. However, the detector device is meant to indicate the collection of parts that actually detect. Whether or not it comprises a number of camera's or the like, they are considered one device here.

Particular embodiments are described in the dependent claims.

In particular, the collimator comprises a wall with a cross-sectional profile in the shape of one of a ring and a regular polygon, in each case having a second axis of rotational symmetry preferably, but not necessarily coinciding with the first axis. This allows pinholes to be positioned around all of the focus volume, to thereby collect full angular information of the object to be imaged, and image count is highest. Herein, "ring" comprises "stack of rings" as well as "cylinder", as the latter is nothing more than a very thick ring. Furthermore, because of the regular shape of the wall, reconstruction algorithms are less complex. Note that the rotational symmetry of the collimator relates to the wall, and not including the pinholes, which can show different fields-of-view, as explained above. Also, since the shape of the object space need not correspond to the shape of the collimator (wall), e.g. due to a tube or other part for delimiting the object space, mounted within the collimator, the types of rotational symmetry for collimator (wall) and object space need not be the same, although their respective axes of symmetry preferably are, though not necessarily. Furthermore, it is pointed out that the collimator need not be rotationally symmetric. In such a case, the second axis is taken to extend longitudinally, through the centre of gravity of a cross-section of the collimator.

In embodiments, the collimator is mounted rotatably around an axis of rotation that extends parallel to the first axis and intersects the object space, and that preferably coincides with the first axis. This allows scanning of the focus volume through the object space, and in some cases also collecting even more angular information of a region of interest in the object. This will be elucidated below. In the case of an axis of rotation not coinciding with the first axis, the collimator will wobble around said first axis. Nevertheless, image reconstruction is still possible, and the specific orbit may be useful when imaging certain objects. However, when the axis of rotation coincides with the first axis, a simple rotation results, and image reconstruction is less complex. For convenience, below we will only discuss this latter situation, although the former is nowhere excluded.

In other embodiments, the collimator comprises a wall extending over least 180° but less than 360° around the focus volume, the wall being rotatable around the first axis. This represents an open collimator, like a C- or U-shaped collimator. This allows e.g. relatively wide objects to be imaged. It also uses less material for pinhole wall, and optionally for the detector. Herein, "around the focus volume" in particular means "around a center of gravity of the focus volume". Furthermore, "at least 180°" preferably means "at least 180° plus the opening angle of the pinholes that are at the ends of the collimator. In this way, substantially full 180° angular information can be obtained. The rotatability allows further angular information to be obtained, as well as scanning, which will be explained further below.

In this application, the "object space" need not be a tangible part, like for example a tube of a radiation transparent plastic or carbon fiber. However, the object space may be considered to be a volume in which the object to be imaged can be placed. This volume could be the inside of a closed collimator, wherein "closed" means "continuous around an opening". In its most specific form, it is a box that can be opened and closed for positioning an object in the inside of the box. Here, it is abundantly clear what the object space is, although it is neither tangible. On the other hand, in the case of a collimator that does not extend completely around the object space, and thus has openings in it, the object space is delimited by the collimator and by (imaginary) planes covering the openings. Similarly, if the collimator is not even "continuous around an opening", such as a C-shaped collimator, but is rotatable, the object space can also indicate the space which is delimited by the collimator when it is (fully) rotated, if necessary covering remaining openings by (imaginary) planes. It is noted that the object space may similarly be delimited by an additional part, such as the above mentioned tube, positioned between the collimator and the object to be imaged. However, in such a case it is possible to take an additional part that is rotationally non-symmetrical, while of course the space delimited by either the ring or polygon collimator, or the rotatable collimator would be rotationally symmetrical. In the later case, the object space is to be taken as the maximum available object space, neglecting said additional part. In all of the above cases, it will be clear what is meant with the object space.

The inventive concept behind the alternatives is the same: there is an object space with a symmetry that is either derivable from the rotationally symmetrical shape of the collimator, or an object space as described by the collimator when rotated around an axis and thus also with a rotational symmetry, and there is a focus volume that is positioned eccentrically, or asymmetrically, with respect to that object space.

In particular embodiments, the collimator is rotatable over at least 20° around the first axis. This relatively small rotational angle may suffice when imaging a region of interest that is not substantially larger than the focus volume, for only little additional angular information will do in such case. An example is a heart, which is a relatively small organ positioned relatively eccentrically. More particularly, the collimator is rotatable over at least 180°, or preferably 180° plus the (average) opening angle of the pinholes. This suffices to scan through a maximum part of the object to be imaged, especially if it is possible to image the object after rotating the object 180°, since rescanning the object then comes down to a full 360° rotation. Most particularly, the collimator is rotatable over 360°. This of course allows a full scan without having to turn the object, which turning is not always possible or advisable, such as with living objects.

In advantageous embodiments, the device further comprises a collimator rotator, for automatically rotating the collimator. Although it is possible to rotate the collimator by hand, through a suitable mechanical coupling, it is advantageous (more precise, less error prone etc.) if such rotation may be performed automatically by a dedicated collimator rotator. In such case it is important to store the angle of rotation as a function of (scanning) time, in order to be able to correlate detector counts and time.

The detector device, having a detector surface and being sensitive to said high energy radiation, and extending at least partially around said object space, may also be rotatable. Thereto, the detector would be coupled to the collimator, allowing simultaneous rotation. The detector could be concentrical to the collimator(s). Preferably, the detectors comprise detector surfaces arranged on a, preferably regular, polygon, such as a triangle (cfr. the U-SPECT designs), a square box or e.g. an octagon. Having multiple but flat detector surface allows simpler detector production and image reconstruction, although rounded detector surfaces are not excluded. Furthermore, the detector extending at least partially around the object space, preferably in a complete loop, indicates that the detector is preferably arranged around the object space as seen in a plane perpendicular to the first axis, such as in a ring or (regular) polygon, without having to extend on all sides of the object space. Furthermore, "loop" is not intended to be limited to a ring.

According to the invention, the focus volume is positioned shifted with respect to the first axis. In embodiments, it is shifted over a non-zero distance d such that the first axis intersects the focus volume. In particular, the distance d is between 10% and 100% of the radius of the focus volume in a radial direction, preferably between 50% and 100% of said radius, and in particular such that the axis of rotation is substantially a tangent to the focus volume.

With the above mentioned distances, rotating the collimator shifts the focus volume through a contiguous region of interest in the object to be scanned, without a part in the middle that has not been scanned by the focus volume, although even in such a situation it is possible to obtain complete data.. This design allows scanning of a large part of the object without shifting it. This is one of the very important advantages of the present invention, because this also means that a relatively larger part of the object space is imageable. A theoretical (though practically not quite attainable) limit relates to a focus volume having a diameter that is half the diameter of the object space, and being positioned just touching the outer limit of the object space. Rotating this focus volume would result in scanning virtually 100% of the object in the object space, which is impossible with the prior art devices.

In other very expedient embodiments, the focus volume is positioned shifted with respect to the first axis over a distance d that is larger than the radius of the focus volume in a radial direction. This is particularly useful if the region of interest in the object to be imaged lies (very) eccentrically. Herein, use is often made of a focus volume that is smaller than half the diameter of the object space. This allows tailoring of the imaging, with size and position of focus volume, to the desired scanning region in the object. Examples are imaging of tumours in skin or ribs. In fact, these embodiments allow very great versatility, combined with great selectivity due to the relatively small focus volume.

Herein, as mentioned above, the position of the focus volume relates to the position of its center of gravity. In cases when the focus volume has a rotationally symmetrical cross-section, it is of course the center. In other cases the geometrical center of gravity is meant. Furthermore, the radial direction is the directing connecting the centre (of gravity) of the focus volume with the first axis, while the radius of the focus volume is the extent measured in said radial direction and starting from the center (of gravity) of the focus volume.

The above mentioned limiting situation is attainable due to the required (full) opening angle being almost 180°, which is of course impossible. In practice, in order to have sufficient definition from the pinhole, a smaller opening angle is necessary, thus positioning the focus volume at a certain distance from the limits of the object space.

In favourable embodiments, the device further comprises an object carrier for carrying the object to be imaged, wherein the object carrier is moveable in at least one and preferably two directions perpendicular to the axis of rotation, more preferably by means of a carrier mover means. This not only allows shifting of the object without rotating the collimator, which simply scans the focus volume through the object in another way. It also allows a combination of shifting the object and rotation of the collimator. Of course, shifting of the carrier mover means along the first axis is deemed a standard implicit possibility, in order to bring the object in the object space, although there are devices in which the object is moved into the object space from the side.

The above described embodiment wherein a relatively small focus volume that is positioned more than its own radius away from the axis of rotation, and having the possibility to shift the object in three dimensions provides extreme advantages, in that it shows great selectivity, due to the small focus which can be moved through virtually all of the object. Furthermore, sensitivity and resolution are increased due to the smaller average distance between pinholes and focus volume, and use of the object space is more efficient, which means that a smaller, lighter and cheaper design is possible.

In particular, the carrier mover means and the collimator rotator are designed and arranged such that the shifting of the object and the rotating of the collimator are coupled to keep the center of the focus volume substantially fixed relative to the object, although the coupling may be stepwise. In other words, if the collimator is rotated and the focus volume thus moves in the object space, the carrier mover means keeps the focus volume substantially fixed to the object, apart from a rotation of the focus volume around its center. This allows not only further angular information to be obtained. It also allows optimum positioning of the collimator with respect to the region of interest. Suppose, for example, that a region of interest is scanned with the collimator in a first position. Image reconstruction reveals that an interesting structure, such as a tumour, is found in the focus volume, but opposite the pinholes closest to the focus volume. It could then be advantageous to rotate the collimator such that now the pinholes closest to the focus volume are also closest to the structure of interest. And in order to keep the focus volume in the same place with respect to the region of interest, the object should be shifted accordingly.

With the present device, and as explained above, it becomes possible to scan the focus volume through the object without at all shifting the object, simply because the focus volume is eccentric with respect to the axis of rotation. Therefore, in many cases, less object space is needed, or a correspondingly smaller collimator, while still being able to scan through the whole body. Contrarily, in prior art devices, any object space needed for a shift to the left necessarily entails a similar amount of object space for the corresponding shift to the right, for scanning through the complete object. Since this "similar amount" to the opposite side is not (always) necessary in the present device, the object space as a whole can be made smaller, as compared to the prior art device, for scanning of objects of the same size. Therefore, in the device according to the present invention, some pinholes are closer to the object or focus volume, as compared to the prior art design, but the other pinholes, though further away than the nearest pinholes, are not further away from the focus volume in an absolute sense than in the prior art device. All this ensures a more efficient use of space, therefore all of the above mentioned advantages, but also, for scanning a certain object size, a smaller possible device, which greatly reduces weight and cost. If the size is kept the same, a higher sensitivity and/or image resolution and/or flexibility when imaging is achievable.
Herein, it is noted that in the prior art device, in order to image an object that had a diameter D that is larger than the diameter of the focus volume by an amount A, the object had to be shifted over a distance 1/2x A to the left, and also over a distance 1/2 x A to the right. Contrarily, in the present device, designs are possible in which, with the same dimensions of the focus volume, no shift at all is necessary, as long as A is smaller than the radial diameter of the focus volume minus twice the displacement of the focus volume with respect to the first axis. In any case, the distance over which the object is to be displaced will be smaller in present devices than in prior art devices, due to the added displacement of the focus volume when rotating the collimator.

In advantageous embodiments, the collimator has at least four pinholes, preferably at least ten pinholes. This ensures not only sufficient angle information and sensitivity of the device, but also a limited complexity. The possible number of pinholes is of course not limited. The pinholes can be arranged in a single ring around the object space, but preferably they are arranged in a three-dimensional structure, such as a stack of rings or a helix, around the object space. Still, the pinholes are focused onto the object space. This configuration allows even more angular information to be obtained. The number and arrangement of the pinholes could for example be 75, cfr. the prior art devices U-SPECT-I and -II with 75 individual pinholes, arranged as five rings with 15 pinholes each.

In some embodiments, the collimator has a collimator body formed as a ring, which allows optimum use of the object space, due to the rotation of the collimator. In another embodiment, the collimator is constructed of a plurality of ring parts or flat plates, connected to form a circumferential collimator body, such as a, preferably regular, polygon. Herein, there are corner parts in the object space, which are imaged incompletely or not at all, especially when the collimator is rotated (thereby defining a cylindrical object space). Each such ring part or plate may have at least one pinhole. Herein, a ring part may be a part of a cylinder, but could also have any other curved shape. In the above, a collimator can comprise a plurality of parts, either connected or not.

Note that further details, such as of a movability of the object carrier, graphical user interfaces, additional so-called framing plates between the collimator and the detector, and so on, are generally known in the art and may be included where desired. Details may be found in e.g. prior patent devices and applications by applicant, such as the already mentioned USPECT-II and WO2007/105942, and US 7,145,153..

In a very expedient embodiment of the invention, the pinholes in the collimator are arranged such that their individual fields-of-view provide two distinct focus volumes, each located eccentrically with respect to said first axis. That is to say, a first collection of pinholes focuses on a first focus volume, while a second collection of pinholes focuses a second focus volume. Advantageously, though not necessarily, each collection comprises neighbouring pinholes. Especially since the focus volumes are located eccentrically, there will be enough space on the collimator for pinholes to be positioned such that for each focus volume full 180° angular information can be obtained. A very big advantage of having two eccentric focus volumes is that the average distance of the collimator to the corresponding focus volume is much smaller, relatively speaking. This ensures an increased sensitivity and resolution. Furthermore, two positions in an object can be imaged at the same time. In particular with two substantially symmetrically positioned eccentric parts in an object, this is already very useful. An example is the kidneys in a an animal or human. Note that it is possible, in some cases, to provide even more than two focus volumes, in particular three focus volumes. This holds in particular for imaging in which it is not necessary to obtain complete data, such as for sparse objects.

In view of the above, it is to be noted that wherever in the introductory part before the preceding paragraph "all pinholes" were said to have a certain property, this always related to all pinholes that focused onto the same focus. If there is only one focus, then all pinholes are meant, but if there are n focus volumes, then only a subcollection of pinholes, that focus onto a particular focus volume, is meant.

In a particular embodiment, the focus volumes are completely separate. This means that image reconstruction of the image for a first focus volume is largely, though not completely, independent of image reconstruction of a second focus volume. In an alternative embodiment, the focus volumes partly overlap. In the latter case, it could be advantageous if the pinholes of one of the focus volumes could be shut during imaging with those of the other focus volume, and vice versa. This allows completely independent imaging, while retaining the advantage of versatility and small distance.

In both the above cases, i.e. overlapping or non-overlapping focus volumes, it is advantageous for the collimator to be rotatable, since this reduces scanning times by at least a factor of 0.5.

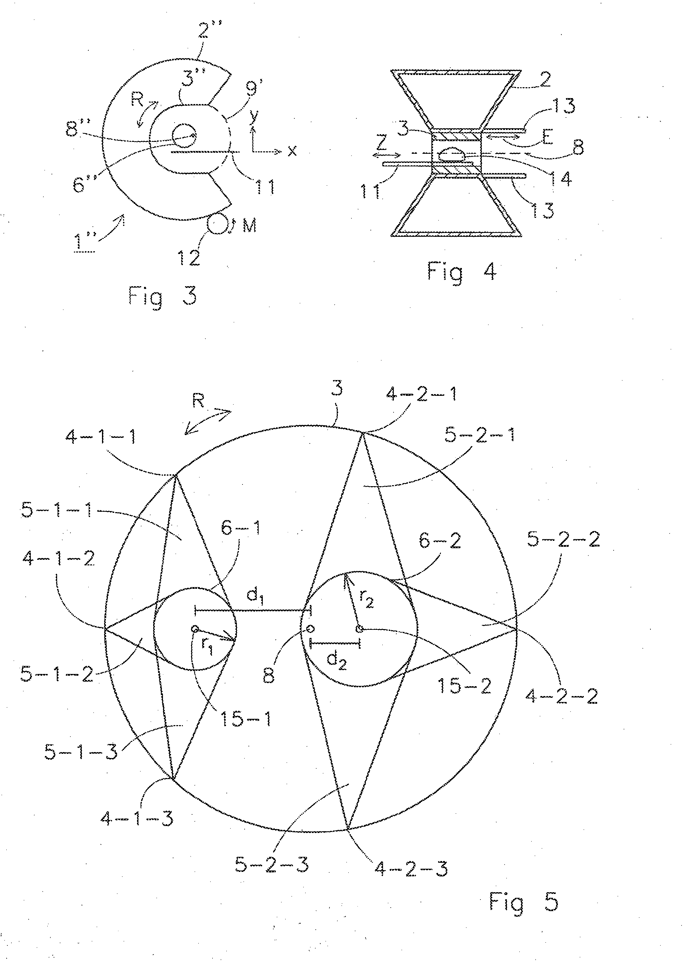

In embodiments, the focus volumes are positioned shifted with respective to the first axis over mutually different distances. Of course, this allows simultaneous imaging of two mutually asymmetric positions. But more importantly, if the collimator is rotatable, it allows a larger portion of the object to be scanned, as will be elucidated with reference to in particular Figure 5 of the drawings.

A particularly advantageous device according to the invention is a single photon emission computed tomography scanner, or so-called SPECT scanner.

Advantageously, the collimator is exchangeable. Of course, this does not relate to the complex action of removing and disconnecting the collimator, which is trivial, but rather to a design for easy exchange. This could advantageously relate to a collimator mover means for moving the collimator away from around the object space, such as to out of the device, or into a holder or container, while moving another collimator with a different design in the place of the first mentioned collimator. Of course, such exchange could also be carried out manually, without a collimator mover means. Such a device allows for example a two-step imaging, such as a first general scan in a first scanning region, followed by a second scan in an interesting part of the first scanning region, requiring a collimator with a smaller focus volume, or with a more eccentric focus volume or the like.

Generally speaking, the advantages as discussed for the detection device according to the invention hold for a collimator with similar features. Hence, each and every advantageous feature with respect to the collimator of the device according to the invention also holds as advantageous for the collimator itself, for example the collimator with two focus volumes, as described above. Such a collimator could for example be built into an existing high energy radiation imaging device, which would then provide the same advantages as for the device according to the present invention.

The invention will now be explained by means of non-limiting embodiments, with reference to the drawings in which:
Figure 1 diagrammatically shows an imaging device 1 according to the invention;
Figure 2 diagrammatically shows another embodiment of a device 1' according to the invention;
Figure 3 diagrammatically shows another embodiment of a device 1" according to the invention;
Figure 4 diagrammatically shows another embodiment of the device according to the invention, in a cross-sectional view; and
Figure 5 diagrammatically shows an advantageous embodiment of a collimator 3.

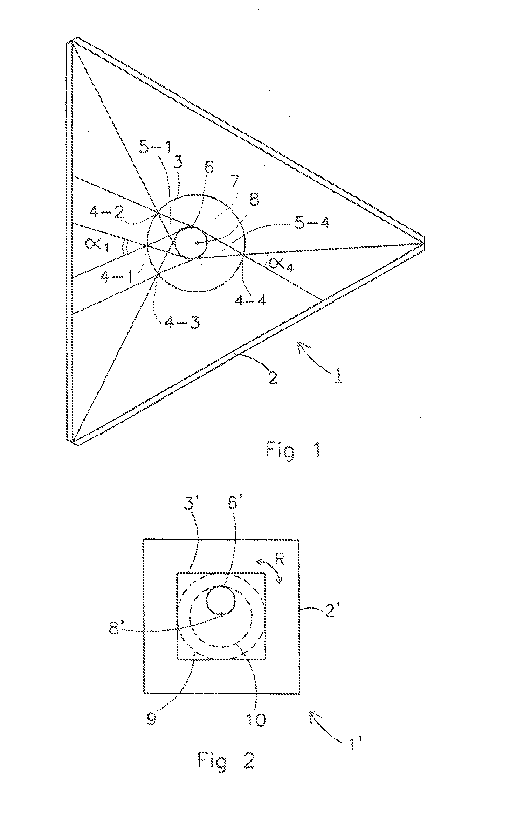

Figure 1 diagrammatically shows an imaging device 1 according to the invention. The device 1 comprises a detector 2, a collimator 3 with pinholes 4-1, 4-2, ..., each having a field-of-view 5-1, 5-2, ..., together defining a focus volume 6, sometimes called a central field-of-view (CFOV). An object space is designated 7, while the first axis, of rotational symmetry, is designated 8.

The detector 2 is shown in the form of a triangular constellation of three detector plates, each e.g. comprising a scintillation device for detecting high energy photons, such as gamma photons. Other detector shapes are possible, such as the ones described below.

The collimator 3, shown only very diagrammatically, is in this case a circular ring with a plurality of pinholes 4-1, 4-2, ..., of which only four are shown here. Each pinhole has a field-of-view 5-1, 5-2, ..., that it can image onto the detector 2. The volume where all individual fields-of-view overlap forms the focus volume 6. This volume provides full angular information for image reconstruction, since it is seen from all directions, i.e. from an angular range of more than 180°.

Within the collimator 3, there is an object space 7, in which an object to be imaged can be positioned. It has an axis of symmetry that naturally coincides with the axis of symmetry of the collimator, which axis of (rotational) symmetry is designated as the first axis 8. The focus volume 6 is positioned eccentrically with respect to the first axis 8. This provides the advantages as mentioned in the introductory part, such as a smaller average distance between the pinholes and the focus volume, allowing increased sensitivity and resolution.

It is noted that the opening angle α₁ of pinhole 4-1, which is closest to the focus volume 6, is in this case, of a circular focus volume, larger than the opening angle α₄ of pinhole 5-4, which is furthest away from the focus volume 6. This holds in case of a circular focus volume 6. Note that other shapes for the focus volume are possible, in each case requiring adaptation of position and/or opening angle of the pinholes.

The device 1 shown is ideally suited for scanning an eccentric part of an object to be imaged, such as an arm or a heart in a human. Since it is almost always possible to move an object into the object space 7 from either side, each of a left arm or a right arm can be scanned with the device 1 of Figure 1. Note herein that "scanning" and "imaging" are used interchangeably herein, i.e. "scanning" does not necessarily entail a moving of the object.

Figure 2 diagrammatically shows another embodiment of a device 1' according to the invention. Herein, as in the rest of the drawings, similar parts are designated with the same cardinal numbers, whether or not primed etc.

Thus, the device 1' comprises a detector 2' and a collimator 3', rotatable in the direction of arrows R and with a focus volume 6', and a first axis 8'. The dashed line 9 indicates the limits of an object space when the collimator 3' is rotated, while 10 indicates the outer limit of a volume that can be scanned by the focus volume 6' when the collimator 3' is rotated.

The collimator 3' and the detector 2' shown here are both polygons, in this case with a square cross-section. These are relatively easily produced.

Strictly speaking, the maximum object space is all the space within the collimator 3'. However, in the embodiment shown, the collimator 3' is rotatable around the first axis 8' in the direction of arrows R. This limits the boundaries of the useful object space as the dashed circle 9. This rotation also shifts the focus volume 6' through the object space, such that a total volume bounded by dashed circle 10 can be imaged. That total volume is a relatively large fraction of the total usable object space. This is due to the fact that the focus volume 6' has been shifted with respect to the first axis 8' such that the latter is a tangent to the former. This ensures a maximum scannable volume without having to shift the object (not shown here). On the other hand, positioning the object to be scanned in a corner of the object space allows it to be positioned at the greatest possible distance of the first axis, and thereby allows the largest possible scanning range in the object, albeit without additional rotation.

Figure 3 diagrammatically shows another embodiment of a device 1" according to the invention. Herein, 11 designates an object carrier such as a bed, while 12 designates a rotating device. The bed 11 is movable in (at least) the directions indicated x and y, and often also along the first axis 8". The rotating device 12 is for rotating the collimator 3", optionally together with the detector 2", in the direction of arrows R, by itself rotating in the direction of arrows M.

Note that the detector 2" and the collimator 3" do not form a closed loop around the focus volume 6". This allows for example a much larger object, that is not shown here but is provided on the bed 11, to be imaged, by inserting it from the side, along the direction designated x. This design is very useful for high magnification scanning of very eccentric parts of an object, such as an arm or ear of a human. Full 180° angular information is obtainable, while even more information can be obtained by rotating the collimator 3" by means of the rotating device 12. The latter is only possible when the object to be imaged is not too large to hinder the rotation. The maximum available size is denoted again by the dashed line 9'.

The moveability of the bed 11, by non-shown carrier mover means such as pneumatic cylinders or any other drive mechanism, in the directions x, y and/or along the first axis 8", allows the following advantageous scanning scheme. First, the object is positioned with respect to the focus volume such that the center (of gravity) of the latter is in a certain position X in the object. Then, by rotating the focus volume, and at the same time adjusting the x-y position of the object, it can be brought about that the center of the focus volume 6" stays in the same position X in the object. This allows the region of interest in the object, around the position X, to be imaged under more possible angles, allowing a better resolution and/or sensitivity. Such moving of the carrier 11 and the collimator 3" is advantageously coupled and performed automatically, though a manual action is possible. Of course, one can also move the object differently with respect to the focus volume, in order to scan the latter through the object.

Figure 4 diagrammatically shows another embodiment of the device according to the invention, in a cross-sectional view.

Shown here is a guiding system 13 with rails, for moving the collimator 3 in the direction of the arrows E, in order to exchange said collimator for another collimator of different design. This may be useful when a different part of an object 14 on the bed 11 should be imaged, or with a different resolution and so on.

To this end, a plurality of different collimators may be provided, together with the rest of the device, as a kit of parts. Such collimators may be stored separate from the rest of the device, or in a revolver etc., all depending e.g. on the size and weight of the various collimators.

Figure 5 diagrammatically shows an advantageous embodiment of a collimator 3.

The collimator 3 has two different focus volumes 6-1 and 6-2, arising from the respective overlap of individual fields-of-view 5-1-1, 5-1-2, ... and 5-2-1, 5-2-2, ..., of respective pinholes 4-1-1, 4-1-2, ..., and 4-2-1, 4-2-2, ..., in each case only showing three pinholes. Of course, a much larger number of pinholes will also be useful. Note that 180° angular information can be obtained with each focus volume rightaway.

A very big advantage of this collimator 3 is that the average distance between the pinholes and the corresponding focus volume is very small, and that it allows extremely versatile imaging. This all depends on the position and size of the respective focus volumes 6-1 and 6-2. A number of cases will be discussed, starting with one shown.

In the case of Figure 5, the focus volume 6-1 is positioned on one side of the first axis 8, at a distance d1, and it has a radius r1. The second focus volume is positioned diametrically opposite, at a distance d2, and it has a radius r2, slightly larger than d2. The first axis 8 is thus just within the second focus volume 6-2. Furthermore, the distance d2 + r2 is larger than the distance d1 - r1. This means that the two focus volumes together will scan through a volume that is bounded by a circle having a radius d1 + r1, when the collimator 3 is rotated in the direction of arrows R over 360°. This is extremely useful, as a focus volume closer to the collimator 3, in this case focus volume 6-1, will often be relatively smaller, due to constraints on the maximum opening angle of the nearest pinhole field-of-view, in this case field 4-1-2 of pinhole 4-1-2. By then selecting a larger focus volume at the opposite side, where the constraint on the corresponding field-of-view 5-2-2 of pinhole 4-2-2 is less stringent, the total radius of the object space within the collimator can be divided up between the two focus volumes. A useful, though in no way limiting, ratio is 1:2. With this embodiment, an extremely large part of the volume (or object space) can be scanned/imaged. Note that the focus volumes 6-1 and 6-2 need not be positioned exactly opposite to each other.

In another embodiment, both focus volumes 6-1 and 6-2 have the same size and distance to the first axis 8, but are positioned opposite to each other with respect to the first axis 8. In this case, optimum use can be made of the fact that the distance between pinholes and focus volume is small. Furthermore, for a complete scan, the collimator need only be rotated over 180°. Therefore, the sampling time will be much shorter, such as about half that for a similar known device with only one centered focus volume, as the average pinhole-focus volume distance is smaller, ensuring a higher sensitivity and thus less time needed for taking an image, while also the rotation time *per se* is halved.

It will be obvious that other advantageous combinations of sizes and positions of the focus volumes can be found. Similarly, all of the above description of certain embodiments is not intended to be limiting. The scope of the invention is given by the appended claims.

## Claims

1. A focused pinhole high energy radiation imaging device (1), comprising:
- an object space (7) for receiving an object to be imaged by means of high energy radiation, and having a first axis (8), that extends longitudinally through a center of gravity of a cross-section of the object space (7) and is preferably an axis of rotational symmetry,
- a detector device (2), having at least one detector surface and being sensitive to said high energy radiation, and extending at least partially, preferably in a complete loop, around said object space,
- a focused pinhole collimator (3) with a focus volume (6), and provided between the at least one detector surface and the object space (7),
**characterized in that** the collimator (3) is designed and arranged in such a way that the focus volume (6) is located eccentrically with respect to said first axis (8).

2. The device according to claim 1, wherein the collimator (3) comprises a wall with a cross-sectional profile in the shape of one of a ring and a regular polygon, in each case having a second axis of rotational symmetry coinciding with the first axis.

3. The device according to claim 2, wherein the collimator (3) is mounted rotatably around an axis of rotation that extends parallel to the first axis (8) and intersects the object space (7), and that preferably coincides with the first axis (8).

4. The device according to claim 1, wherein the collimator (3) comprises a wall extending over least 180° but less than 360° around the focus volume (6), the wall being rotatable around the first axis (8).

5. The device according to claim 3 or 4, wherein the collimator (3) is rotatable over at least 20° around the first axis (8), preferably over at least 180°, and more preferably over 360°.

6. The device according to any of claims 3-5, further comprising a collimator rotator, for automatically rotating the collimator (3).

7. The device according to any preceding claim, wherein the focus volume (6) is positioned shifted with respect to the first axis (8) over a non-zero distance d such that the first axis (8) intersects the focus volume (6), and in particular wherein the distance d is between 10% and 100% of the radius of the focus volume (6) in a radial direction, preferably between 50% and 100% of said radius, and more in particular such that the axis of rotation is substantially a tangent to the focus volume (6).

8. The device according to any of claims 1-6, wherein the focus volume (6) is positioned shifted with respect to the first axis (8) over a non-zero distance d that is larger than the radius of the focus volume (6) in a radial direction.

9. The device according to any preceding claim, further comprising an object carrier (11) for carrying the object to be imaged, wherein the object carrier (11) is moveable in at least one and preferably two directions perpendicular to the axis of rotation, more preferably by means of a carrier mover means.

10. The device according to any preceding claim, wherein the pinholes (4-1-1, 4-1-2, 4-1-3, 4-2-1, 4-2-2, 4-2-3) in the collimator (3) are arranged such that their individual fields-of-view (5-1-1, 5-1-2, 5-1-3, 5-2-1, 5-2-2, 5-2-3) provide two different focus volumes (6-1, 6-2), each located eccentrically with respect to said first axis (8).

11. The device according to claim 10, wherein the focus volumes (6-1, 6-2) are completely separate.

12. The device according to claim 10, wherein the focus volumes (6-1, 6-2) partly overlap, advantageously wherein the pinholes of one of the focus volumes (6-1, 6-2) are shuttable during imaging with the pinholes of the other focus volume, and vice versa.

13. The device according to any of claims 10-12, wherein the focus volumes (6-1, 6-2) are positioned shifted with respective to the first axis (8) over mutually different distances.

14. Method for imaging an object by means of high energy radiation using a focused pinhole high energy radiation imaging device according to any of claims 1-13.

## Patentansprüche

1. Fokussierte Pinhole-Bildgebungsvorrichtung (1), die auf Basis von energiereicher Strahlung arbeitet, mit:
- einem Objektraum (7) zum Aufnehmen eines mittels energiereicher Strahlung abzubildenden Objekts und mit einer ersten Achse (8), die sich in Längsrichtung durch ein Gravitationszentrum eines Querschnitts des Objektraums (7) erstreckt und vorzugsweise eine Rotationssymmetrieachse ist,
- einer Detektoreinrichtung (2) mit wenigstens einer Detektorfläche und empfindlich für energiereiche Strahlung, und die sich wenigstens teilweise, vorzugsweise in einer vollständigen Schleife, um dem Objektraum herum erstreckt,
- einem fokussierten Pinhole-Kollimator mit einem Fokusvolumen (6), wobei der Kollimator zwischen der wenigstens einen Detektorfläche und dem Objektraum (7) angeordnet ist,
**dadurch gekennzeichnet, dass** der Kollimator (3) in der Weise gestaltet und angeordnet ist, dass das Fokusvolumen (6) in Bezug zu der ersten Achse (8) exzentrisch angeordnet ist.

2. Vorrichtung nach Anspruch 1, in welcher der Kollimator (3) eine Wand mit einem Querschnittsprofil in Form eines Rings oder regelmäßigen Polygons umfasst, wobei dieser in jedem Falle eine zweite Rotationssymmetrieachse aufweist, die mit der ersten Achse zusammenfällt.

3. Vorrichtung nach Anspruch 2, in welcher der Kollimator (3) drehbar um eine Rotationachse angebracht ist, die sich parallel zu der ersten Achse (8) erstreckt und den Objektraum (7) schneidet und die vorzugsweise mit der ersten Achse (8) zusammenfällt.

4. Vorrichtung nach Anspruch 1, in welcher der Kollimator (3) eine sich über wenigstens 180° Grad, aber weniger als 360° Grad, um das Fokusvolumen (6) erstreckende Wand umfasst, wobei die Wand um die erste Achse (8) drehbar ist.

5. Vorrichtung nach Anspruch 3 oder 4, in welcher der Kollimator (3) über wenigstens 20° Grad, vorzugsweise wenigstens über 180° Grad und ganz bevorzugt über 360° Grad um die erste Achse (8) drehbar ist.

6. Vorrichtung nach einem der Ansprüche 3 bis 5, ferner mit einem Kollimator-Rotator zum automatischen Rotieren des Kollimators (3).

7. Vorrichtung nach einem vorstehenden Anspruch, in welcher das Fokusvolumen (6) in Bezug zu der ersten Achse (8) über eine Distanz b ungleich Null versetzt positioniert ist, derart, dass die erste Achse (8) das Fokusvolumen (6) schneidet, und insbesondere in welcher die Strecke d zwischen 10% und 100% des Radius des Fokusvolumens (6) in einer radialen Richtung, vorzugsweise zwischen 50% und 100% des Radius beträgt, und ganz bevorzugt derart, dass die Rotationsachse im Wesentlichen eine Tangente des Fokusvolumens (6) ist.

8. Vorrichtung nach einem der Ansprüche 1 bis 6, in welcher das Fokusvolumen (6) in Bezug zu der ersten Achse (8) über eine Strecke d ungleich Null versetzt positioniert ist, die größer ist als der Radius des Fokusvolumens (6) in radialer Richtung.

9. Vorrichtung nach einem vorhergehenden Anspruch, ferner mit einem Objektträger (11) zum Tragen des abzubildenden Objekts, in welcher der Objektträger (11) in wenigstens einer und vorzugsweise in zwei Richtungen senkrecht zur Rotationsachse bewegbar ist, vorzugsweise mit Hilfe einer Träger-Bewegungseinrichtung.

10. Vorrichtung nach einem vorhergehenden Anspruch, in welcher die Pinholes (4-1-1, 4-1-2, 4-1-3, 4-2-1, 4-2-2, 4-2-3) in dem Kollimator (3) derart angeordnet sind, dass ihre individuellen Sichtfelder (5-1-1, 5-1-2, 5-1-3, 5-2-1, 5-2-2, 5-2-3) zwei unterschiedliche Fokusvolumina (6-1, 6-2) liefern, die jeweils exzentrisch in Bezug zu der ersten Achse (8) angeordnet sind.

11. Vorrichtung nach Anspruch 10, in welcher die Fokusvolumina (6-1, 6-2) vollständig separat sind.

12. Vorrichtung nach Anspruch 10, in welcher sich die Fokusvolumina (6-1, 6-2) teilweise überlagern, vorteilhafterweise in welcher die Pinholes eines der Fokusvolumina (6-1, 6-2) während der Bildgebung mit den Pinholes des anderen Fokusvolumen verschließbar sind.

13. Vorrichtung nach einen der Ansprüche 10 bis 12, in welcher die Fokusvolumina (6,1, 6,2) in Bezug zu der ersten Achse (8) über zueinander unterschiedliche Strecken versetzt positioniert sind.

14. Verfahren zum Abbilden eines Objekts auf Basis von energiereicher Strahlung unter Verwendung einer fokussierten Bildgebungsvorrichtung, die auf Basis von energiereicher Strahlung arbeitet, nach einem der Ansprüche 1 bis 13.

## Revendications

1. Dispositif de formation d'image à rayonnement à haute énergie à sténopés focalisés (1), comprenant :
- un espace d'objet (7) pour recevoir un objet dont l'image doit être formée au moyen d'un rayonnement à haute énergie, et ayant un premier axe (8), qui s'étend longitudinalement à travers un centre de gravité d'une section de l'espace d'objet (7) et qui est de préférence un axe de symétrie de rotation,
- un dispositif de détection (2), comportant au moins une surface de détection et sensible au dit rayonnement à haute énergie, et s'étendant au moins partiellement, de préférence en une boucle complète, autour dudit espace d'objet,
- un collimateur à sténopés focalisés (3) avec un volume de focalisation (6), et prévu entre ladite au moins une surface de détection et l'espace d'objet (7),
**caractérisé en ce que** le collimateur (3) est conçu et agencé de manière à ce que le volume de focalisation (6) soit situé de manière excentrique par rapport au dit premier axe (8).

2. Dispositif selon la revendication 1, dans lequel le collimateur (3) comprend une paroi avec un profil en coupe sous la forme de l'un d'une bague et d'un polygone régulier, ayant dans chaque cas un deuxième axe de symétrie de rotation coïncidant avec le premier axe.

3. Dispositif selon la revendication 2, dans lequel le collimateur (3) est monté en rotation autour d'un axe de rotation qui s'étend parallèlement au premier axe (8) et qui croise l'espace d'objet (7), et qui coïncide de préférence avec le premier axe (8).

4. Dispositif selon la revendication 1, dans lequel le collimateur (3) comprend une paroi s'étendant sur au moins 180°, mais moins de 360°, autour du volume de focalisation (6), la paroi pouvant tourner autour du premier axe (8).

5. Dispositif selon la revendication 3 ou 4, dans lequel le collimateur (3) peut tourner sur au moins 20° autour du premier axe (8), de préférence sur au moins 180°, et plus préférablement sur 360°.

6. Dispositif selon l'une quelconque des revendications 3 à 5, comprenant en outre un dispositif de rotation de collimateur, pour faire tourner automatiquement le collimateur (3) .

7. Dispositif selon l'une quelconque des revendications précédentes, dans lequel le volume de focalisation (6) est positionné de manière à être décalé par rapport au premier axe (8) sur une distance non nulle d de sorte que le premier axe (8) croise le volume de focalisation (6), et en particulier dans lequel la distance d est entre 10 % et 100 % du rayon du volume de focalisation (6) dans une direction radiale, de préférence entre 50 % et 100 % dudit rayon, et plus particulièrement de sorte que l'axe de rotation soit sensiblement une tangente au volume de focalisation (6).

8. Dispositif selon l'une quelconque des revendications 1 à 6, dans lequel le volume de focalisation (6) est positionné de manière à être décalé par rapport au premier axe (8) sur une distance non nulle d qui est supérieure au rayon du volume de focalisation (6) dans une direction radiale.

9. Dispositif selon l'une quelconque des revendications précédentes, comprenant en outre un support d'objet (11) pour supporter l'objet dont l'image doit être formée, dans lequel le support d'objet (11) peut être déplacé dans au moins une et de préférence deux directions perpendiculaires à l'axe de rotation, plus préférablement à l'aide de moyens de déplacement de support.

10. Dispositif selon l'une quelconque des revendications précédentes, dans lequel les sténopés (4-1-1, 4-1-2, 4-1-3, 4-2-1, 4-2-2, 4-2-3) dans le collimateur (3) sont agencés de sorte que leurs champs de vision individuels (5-1-1, 5-1-2, 5-1-3, 5-2-1, 5-2-2, 5-2-3) fournissent deux volumes de focalisation (6-1, 6-2) différents, situés chacun de manière excentrique par rapport au dit premier axe (8).

11. Dispositif selon la revendication 10, dans lequel les volumes de focalisation (6-1, 6-2) sont complètement séparés.

12. Dispositif selon la revendication 10, dans lequel les volumes de focalisation (6-1, 6-2) se superposent partiellement, avantageusement dans lequel les sténopés de l'un des volumes de focalisation (6-1, 6-2) peuvent être obturés pendant la formation d'image avec les sténopés de l'autre volume de focalisation, et vice versa.

13. Dispositif selon l'une quelconque des revendications 10 à 12, dans lequel les volumes de focalisation (6-1, 6-2) sont positionnés de manière à être décalés par rapport au premier axe (8) sur des distances mutuellement différentes.

14. Procédé pour former l'image d'un objet au moyen d'un rayonnement à haute énergie en utilisant un dispositif de formation d'image à rayonnement à haute énergie à sténopés focalisés selon l'une quelconque des revendications 1 à 13.
